# EUROPEAN PATENT SPECIFICATION

(11) **EP 3 016 980 B1**
(45) Date of publication and mention of the grant of the patent: **06.05.2020**
(21) Application number: 14819422.8
(22) Date of filing: 04.07.2014
(51) Int. Cl.: A61K 47/68, C07K 16/28, A61K 47/50, A61P 35/00

(54) **EGFR ANTIBODY CONJUGATES**
EGFR-ANTIKÖRPERKONJUGATE
CONJUGUÉS D'ANTICORPS ANTI-EGFR

(30) Priority: 05.07.2013 US 201361843113 P; 25.02.2014 US 201461944157 P
(43) Date of publication of application: 11.05.2016
(73) Proprietor: Formation Biologics Inc., Toronto, ON M5G 1L7 (CA)
(72) Inventor: TIKHOMIROV, Ilia Alexandre, Toronto, Ontario M8Z 1P5 (CA)
(74) Representative: Leonard, Thomas Charles
(86) International application number: PCT/CA2014/000543
(87) International publication number: WO 2015/000062

(56) References cited:
- WO-A1-2013/075048
- WO-A1-2014/177771
- WO-A2-2012/058592
- WO-A2-2013/163631
- WO-A2-2015/038984
- US-A1- 2012 156 217
- US-A1- 2012 156 217
- US-A1- 2013 156 796
- US-A1- 2013 156 796
- Yulius Y. Setiady ET AL: "Abstract 5463: Development of a novel antibody-maytansinoid conjugate, IMGN289, for the treatment of EGFR-expressing solid tumors.", Cancer Research, 15 April 2013 (2013-04-15), pages 1-4, XP055344770, Retrieved from the Internet: URL:http://cancerres.aacrjournals.org/cont ent/73/8_Supplement/5463 [retrieved on 2017-02-13]

## Description

### FIELD OF THE INVENTION

The present invention relates to an immunoconjugate that targets EGFR-expressing cancer cell populations and comprises an anti-EGFR antibody having the amino acid sequence of SEQ ID NO: 10 for the light chain and SEQ ID NO: 9 for the heavy chain, conjugated to the microtubule damaging agent maytansinoid DM-1 by the linker succinimidyl 4-(N-maleimidomethyl)-cyclohexane-1-carboxylate (SMCC).

### BACKGROUND TO THE INVENTION

The conjugation of cell-binding proteins, such as antibodies, to potent cell-killing agents to enhance their anti-cancer activity, and provide the so-called "magic bullets" has had mixed clinical results.

US 2013/0156796 describes that EGFR antibody immunoconjugates may be effective in inhibiting tumor cells that have developed EGFR and/or ALK resistance mechanisms.

US 2012/0156217 relates to anti-cancer agents including antibodies and immunoconjugates that bind to EGFR.

Setiady et al., Cancer Res. 2013, 73:5463 relates to an antibody - maytansinoid conjugate, IMGN289, for the treatment of EGFR-expressing solid tumors.

Relative to naked antibodies, immunoconjugates often show enhanced cell-killing potency, and this increases their activity against cancer cells expressing the antibody-targeted antigen. The same increase in potency is also seen, however, in normal cells that express that same antigen. Of particular concern is the increased cytotoxicity against the rapidly proliferating tissues, such as skin. For example, a CD44v6-targeting immunoconjugate consisting of a maytansinoid and CD44v6 antibody was very active against cancer cells but was discontinued because of severe skin toxicities, such as toxic epidermal necrolysis, which occurred as a result of enhanced activity of the immunoconjugate against skin cells also expressing CD44v6 (Tijink et al., Clin Cancer Res, 2006, 12:6064).

Another cell surface protein, epidermal growth factor receptor, or EGFR, is an attractive target for the development of anti-cancer immunoconjugates because of the antigen's expression by many tumors and its rapid internalization. However, because EGFR is also expressed by skin tissues, EGFR-targeting agents, such as the antibodies cetuximab and panitumumab, also show levels of skin toxicities that either demand dose reduction or in some cases are so severe as to warrant discontinuation of treatment.

With immunoconjugates, the toxicity of these antibodies is amplified through conjugation to a potent cell toxin. This exacerbates the problem for antibodies that are already inherently toxic to normal cells. For instance, conjugation with a toxic maytansinoid caused severe toxicity against skin cells when delivered via a CD44v6 antibody. As a result, development of anti-EGFR immunoconjugates based on approved anti-EGFR antibodies has not been pursued because of concerns over enhanced skin toxicity of such immunoconjugates. Alternative strategies are being pursued for the development of anti-EGFR immunoconjugates.

Anti-EGFR immunoconjugates are now being designed specifically to address these safety concerns. These conjugates are based on antibodies that target a mutated but naturally occurring version of EGFR, known as EGFRvIII, or on conformational forms of the EGFR, both of which predominate on tumour cells and not on skin cells (US 7628986, and US 7589180, respectively). For example, anti-EGFR antibody MAb806 is an antibody that targets an EGFR epitope found only on cancer cells, and potentially offers an advantage over the current EGFR antibodies, which all display significant binding to normal organs such as skin in humans. With this specificity, it is recognized that "the most important advantage of MAb 806 compared to current EGFR antibodies, is that MAb 806 can be directly conjugated to cytotoxic agents", an approach not feasible with other EGFR antibodies since the "cytotoxic conjugation would almost certainly induce severe toxicity" (US 7589180). An immunoconjugate comprising EGFR MAb 806 linked to an anti-microtubule payload is currently in phase I clinical testing in patients with advanced solid tumours.

Efforts continue through screening for naked anti-EGFR antibodies, and immunoconjugates thereof, to identify those with partial antagonistic activity against EGFR and reduced activity against keratinocytes (see US 2012/0156217), immunoconjugates based on "masked" anti-EGFR antibodies that are preferentially activated in the tumour microenvironment (WO 2009/025846), and immunoconjugates based on antibodies with medium affinity that preferentially accumulate in the tumor and not normal tissues (WO 2012/100346). All of these strategies are aimed at reducing toxicities toward skin and other organs expressing EGFR, because currently approved anti-EGFR antibodies were deemed unsuitable for development as immunoconjugates.

An object of the present invention is to provide an immunoconjugate useful to treat EGFR+ disease cells including EGFR+ cancer cells and tumours comprising them. Another object of the present invention is to provide a method for potentiating the cytotoxicity of an EGFR antibody toward disease cells selectively. By this method, potentiation of toxicity toward normal cells is essentially avoided.

### SUMMARY OF THE INVENTION

The invention is as defined by the claims. Any subject matter falling outside the scope of the claims is provided for information purposes only. Any references in the description to methods of treatment refer to the compounds, pharmaceutical compositions and medicaments of the present invention for use in a method for treatment of the human or animal body by therapy.

The present invention provides a method useful to potentiate the cytotoxicity of an EGFR antibody on EGFR+ disease cells without potentiating the cytotoxicity thereof on normal EGFR+ cells, the method comprising:
(i) selecting, for conjugation, an EGFR antibody that is a full EGFR antagonist and competes with cetuximab for binding to EGFR;
(ii) selecting, for delivery by the EGFR antibody, an anti-microtubule toxin;
(iii) selecting, for coupling the selected EGFR antibody and the anti-microtubule toxin, a linker; and
producing an immunoconjugate that incorporates the linker between the antibody and the toxin, thereby providing an immunoconjugate having a cytotoxicity that is potentiated against EGFR+ disease cells and essentially not potentiated against EGFR+ keratinocyte cells; wherein the selected EGFR antibody is an EGFR antibody having the amino acid sequence of SEQ ID No. 10 for the light chain and SEQ ID No. 9 for the heavy chain, the selected anti-microtubule toxin is the maytansinoid DM-1 and the selected linker is succinimidyl 4-(N-maleimidomethyl)-cyclohexane-1-carboxylate (SMCC).

The present invention also provides an immunoconjugate comprising (i) an EGFR antibody having the amino acid sequence of SEQ ID No. 10 for the light chain and SEQ ID No. 9 for the heavy chain, and (ii) DM-1 conjugated therewith, by (iii) a linker that is SMCC, the immunoconjugate having a cytotoxic effect relative to a naked form of said antibody that is (1) potentiated with respect to EGFR+ cancer cells, and (2) substantially unaltered with respect to EGFR+ keratinocytes.

The present invention also provides a method for potentiating the effect of an EGFR antibody on EGFR+ disease cells without potentiating the effect thereof on normal EGFR+ cells, comprising linking said EGFR antibody to DM-1 by an SMCC linker, wherein the EGFR antibody has the amino acid sequence of SEQ ID No. 10 for the light chain and SEQ ID No. 9 for the heavy chain.

The present invention also provides an EGFR antibody in a form conjugated with DM-1 via an SMCC linker for use in a method of treating a subject presenting with a tumour that responds to treatment with an EGFR antibody, wherein treatment elicits a EGFR antibody-mediated adverse response by keratinocytes, whereby the tumour response to treatment with the conjugated EFGR antibody is enhanced essentially without enhancing the adverse keratinocyte response to treatment, relative to treatment with naked antibody alone, wherein the EGFR antibody has the amino acid sequence of SEQ ID No. 10 for the light chain and SEQ ID No. 9 for the heavy chain.

The present invention further provides a pharmaceutical composition comprising an immunoconjugate of the invention in an amount cytotoxic to EGFR+ disease cells, and a pharmaceutically acceptable carrier.

The present invention further provides a method for producing an anti-cancer composition, comprising the step of combining a pharmaceutically acceptable carrier, and an immunoconjugate of the invention.

The present invention further provides the immunoconjugate of the invention or the pharmaceutical composition comprising an immunoconjugate of the invention for use in a method of treating cancer.

The present invention draws from the unexpected initial finding that conjugation of the anti-EGFR antibody cetuximab to a toxic maytansinoid through a non-cleavable linker yields an immunoconjugate that displays enhanced cytotoxicity against cancer cells without a corresponding increase in cytotoxicity against skin cells. The inventor demonstrates that the activity of some anti-EGFR antibodies against both cancer and keratinocytes is strongly potentiated by linking to maytansinoids and other cell-killing agents, whereas the activity of maytansinoid-conjugated cetuximab is potentiated only against cancer cells and not against keratinocytes. In these studies, conjugates of cetuximab with cell-killing agents other than anti-microtubule toxins, such as saporin, were found, as expected, to have an attendant and significantly enhanced toxicity toward keratinocytes.

It is further demonstrated that another antibody having full antagonist activity at EGFR, i.e. panitumumab, (not claimed) also demonstrates selective potentiation at EGFR+ cells when conjugated to an anti-microtubule payload such as a maytansinoid, in showing toxicity to EGFR+ cancer cells while sparing EGFR+ normal cells such as keratinocytes.

On the basis of these and other findings herein disclosed, the present invention enables the selection of components essential to yield an immunoconjugate that comprises an EGFR antibody and a toxic payload that potentiates antibody activity toward cancer cells but not toward normal cells such as keratinocytes. Immunoconjugates having this property require the selection of an EGFR antibody that is a full antagonist, a toxin that is an anti-microtubule agent, and a linker that most desirably is not cleavable. By applying these criteria, there is provided an EGFR antibody-based immunoconjugate that has significant therapeutic activity against EGFR+ cancer cells without a corresponding increase in toxicity against EGFR+ normal cells including skin cells such as keratinocytes. Lack of additional potentiation of the toxicity against skin cells is critical because naked EGFR-targeting antibodies are already characterized by high prevalence dermatologic toxicities and it is beneficial not to potentiate these side effects.

Accordingly, in a general aspect, there is provided an immunoconjugate comprising an antibody having full antagonist activity at EGFR, and a toxin conjugated therewith through a non-cleavable linker, the immunoconjugate having a cytotoxic effect relative to a naked form of the antibody that is essentially not potentiated with respect to EGFR+ keratinocytes. The cytotoxic effect of the immunoconjugate with respect to EGFR+ cancer cells desirably is potentiated. The toxic payload desirably is an anti-microtubule toxin.

Also in a general aspect, there is provided a method useful to potentiate the anti-cancer activity of an EGFR antibody without potentiating the effect thereof on normal EGFR+ cells, the method comprising:
(i) selecting, for conjugation, an EGFR antibody that is a full EGFR antagonist and competes with cetuximab for binding to EGFR;
(ii) selecting, for delivery by the EGFR antibody, an anti-microtubule toxin;
(iii) selecting, for coupling the selected EGFR antibody and the anti-microtubule toxin, a linker; and
producing an immunoconjugate that incorporates the linker between the antibody and the toxin, thereby providing an immunoconjugate having a cytotoxicity that is potentiated against EGFR+ disease cells and essentially not potentiated against normal EGFR+ keratinocyte cells.

In one particular aspect of the disclosure, there is provided an immunoconjugate comprising cetuximab and a toxin conjugated therewith, the immunoconjugate having a cytotoxic effect relative to naked cetuximab that is (1) enhanced with respect to EGFR+ cancer cells, and (2) substantially not enhanced with respect to EGFR+ keratinocytes, wherein the immunoconjugate comprises cetuximab and an anti-microtubule toxin such as maytansinoid DM-1 conjugated by a non-cleavable linker. In alternative embodiments of the invention, the cetuximab is an equivalent of cetuximab, such as an EGFR-binding fragment of cetuximab, or an EGFR-binding variant of cetuximab that incorporates one or two or more benign substitutions in the antibody constant region or framework region without affecting the antibody binding to the receptor or antibody conjugate-mediated cell killing. For example, chimeric cetuximab can be further humanized using standard methods to create a more human-like version of the antibody. Alternatively, a fully human anti-EGFR antibody, such as necitumumab also known as IMC-11F8, which is considered to be functionally equivalent to cetuximab can be developed by screening of a human Fab library for an antibody that can bind and strongly inhibit EGFR and competes with cetuximab for receptor binding (Li S., Kussie P., Fergusson KM, Structural basis for EGF receptor inhibition by the therapeutic antibody IMC-11F8. Structure. 2008 Feb;16(2):216-27).

In another particular aspect of the disclosure, there is provided an immunoconjugate comprising panitumumab and a toxin conjugated therewith, the immunoconjugate having a cytotoxic effect relative to naked panitumumab that is (1) enhanced with respect to EGFR+ cancer cells, and (2) substantially unaltered with respect to EGFR+ keratinocytes, wherein the immunoconjugate comprises panitumumab and an anti-microtubule toxin such as maytansinoid DM-1 conjugated by a non-cleavable linker. In alternative embodiments of the disclosure, the panitumumab is an EGFR-binding fragment of panitumumab, or is a variant of panitumumab that incorporates one, two, or more benign substitutions yet maintains EGFR binding and inhibitory characteristics of the panitumumab parent.

In preferred embodiments, conjugation of the antibody and toxin is achieved using a non-cleavable linker. With non-cleavable linkers, release of the cytotoxic payload occurs by intracellular destruction of the drug conjugate by lysosomes. A non-cleavable linker is substantially resistant to acid-induced cleavage, light-induced cleavage, peptidase-induced cleavage, esterase-induced cleavage, or disulfide bond cleavage, whereas cleavable linkers, which can be used optionally but less desirably, are linkers that can be cleaved by one or more of these recited cleaving agents. Examples of such non-cleavable linkers include those that are or can be derived from a haloacetyl-based moiety selected from the group consisting of N-succinimidyl-4-(iodoacetyl)-aminobenzoate (SIAB), N-succinimidyl iodoacetate (SIA), N-succinimidyl bromoacetate (SBA), and N-succinimidyl 3-(bromoacetamido)propionate (SBAP). Alternatively, the non-cleavable linker is or is derived from a maleimido-based moiety selected from the group consisting of N-succinimidyl 4-(maleimidomethyl)cyclohexanecarboxylate (SMCC), N-succinimidyl-4-(N-maleimidomethyl)-cyclohexane- 1 -carboxy-(6-amidocaproate) (LC-SMCC), κ-maleimidoundecanoic acid N-succinimidyl ester (KMUA), γ-maleimidobutyric acid N-succinimidyl ester (GMBS), ε-maleimidcaproic acid N-hydroxysuccinimide ester (EMCS), m-maleimidobenzoyl-N-hydroxysuccinimide ester (MBS), N-(α-maleimidoacetoxy)-succinimide ester (AMAS), succinimidyl-6-(β-maleimidopropionamido)hexanoate (SMPH), N-succinimidyl 4-(p-maleimidophenyl)-butyrate (SMPB), and N-(p-maleimidophenyl)isocyanate (PMPI); another non-cleavable linker is maleimidocaproyl. (See US 2005/0169933; , Yoshitake et al, 101 Eur. J. Biochem. 395-399 (1979); Hashida et al, J. Applied Biochem. 56-63 (1984); and Liu et al, 18 690-697 (1979), and Doronina et al,. Bioconjugate Chem., 2006 Jan-Feb;17(1):114-24 for additional details.)

In a preferred embodiment, conjugation is achieved using a non-cleavable cross-linking reagent as linker such as succinimidyl 4-(N-maleimidomethyl)-cyclohexane-1-carboxylate (SMCC). Other useful forms of non-cleavable linkers include N-Succinimidyl iodoacetate (SIA), sulfo-SMCC, m-maleimidobenzoyl-N-hydroxysuccinimide ester (MBS), sulfo-MBS and succinimidyl-iodoacetate, as described in the literature, which introduce 1-10 reactive groups. (see, Yoshitake et al, 101 Eur. J. Biochem. 395-399 (1979); Hashida et al, J. Applied Biochem. 56-63 (1984); and Liu et al, 18 690-697 (1979)). Particularly useful for linking auristatins as anti-microtubule toxin are the non-cleavable maleimidocaproyl linkers described by Doronina et al, in Bioconjugate Chem., 2006 Jan-Feb; 17(1): 114-24). According to the invention, the linker is SMCC.

In another aspect, there is provided a pharmaceutical composition comprising the present EGFR antibody-based immunoconjugate in an amount cytotoxic to EGFR+ cancer cells, and a pharmaceutically acceptable carrier.

In a further aspect, there is provided the present pharmaceutical composition for use in the treatment of EGFR+ cancer cells. In a related aspect, there is provided the present immunoconjugate in an amount cytotoxic to the EGFR+ cancer cell for use in a method for treating a subject presenting with an EGFR+ cancer cell.

These and other aspects of the present invention are now described in greater detail with reference to the accompanying drawings in which:

### BRIEF DESCRIPTION OF THE DRAWINGS

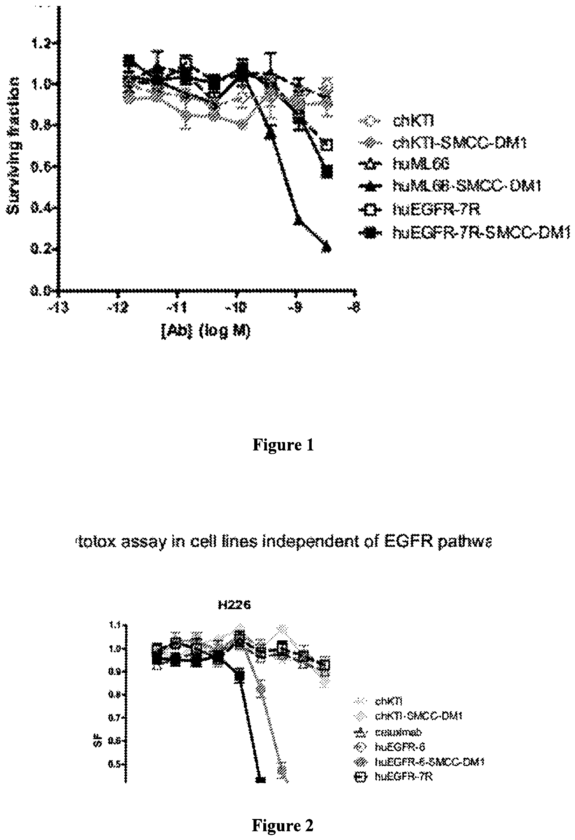

Figure 1 is a graph showing cytotoxic activity against keratinocytes of naked anti-EGFR antibodies and maytansinoid conjugates thereof. About 2-3,000 cells/well were seeded in a flat bottom 96-well tissue culture plate, and incubated with various concentrations of test article in culture media for 5 days at 37°C. Viability of the remaining cells was determined by WST-8 based colorimetric assay.
Figure 2 is a graph showing cytotoxic activity against cancer cell line of naked anti-EGFR antibodies and maytansinoid conjugates thereof. About 2-3,000 cells/well were seeded in a flat bottom 96-well tissue culture plate, and incubated with various concentrations of test article in culture media for 5 days at 37°C. Viability of the remaining cells was determined by WST-8 based colorimetric assay.
Figures 1 and 2 herein are reproductions of figures presented in US 2012/0156217.

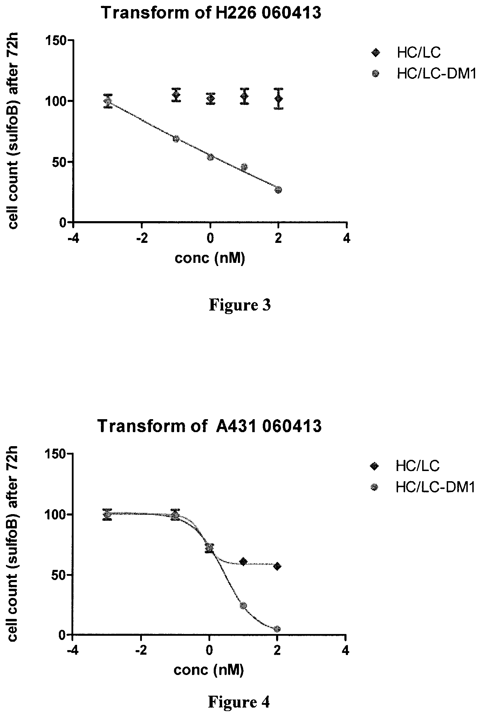

Figure 3 is a graph showing cytotoxic activity against H226 cancer cell line of naked anti-EGFR antibody cetuximab and maytansinoid conjugates thereof. About 2-3,000 cells/well were seeded in a flat bottom 96-well tissue culture plate, and incubated with various concentrations of test article in culture media for 72 h at 37°C. Viability of the remaining cells was determined via alamar blue cell viability assay.
Figure 4 is a graph showing cytotoxic activity against A431 cancer cell line of naked anti-EGFR antibody cetuximab and maytansinoid conjugates thereof. About 2-3,000 cells/well were seeded in a flat bottom 96-well tissue culture plate, and incubated with various concentrations of test article in culture media for 72 h at 37°C. Viability of the remaining cells was determined via alamar blue cell viability assay.

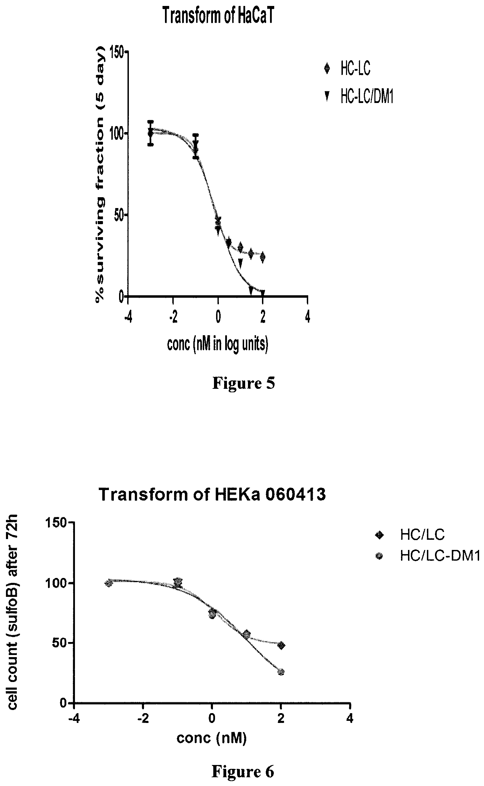

Figure 5 is a graph showing the cytotoxic activity of naked anti-EGFR antibody cetuximab and maytansinoid conjugates thereof against normal keratinocyte cell line HaCaT. About 2-3,000 cells/well were seeded in a flat bottom 96-well tissue culture plate, and incubated with various concentrations of test article in culture media for 5 days at 37°C. Viability of the remaining cells was determined via alamar blue cell viability assay.
Figure 6 is a graph showing the cytotoxic activity of naked anti-EGFR antibody cetuximab and maytansinoid conjugates thereof against primary keratinocytes. 2-3,000 cells/well were seeded in a flat bottom 96-well tissue culture plate, and incubated with various concentrations of test article in culture media for 72 h or 5 days at 37°C. Viability of the remaining cells was determined via alamar blue cell viability assay.

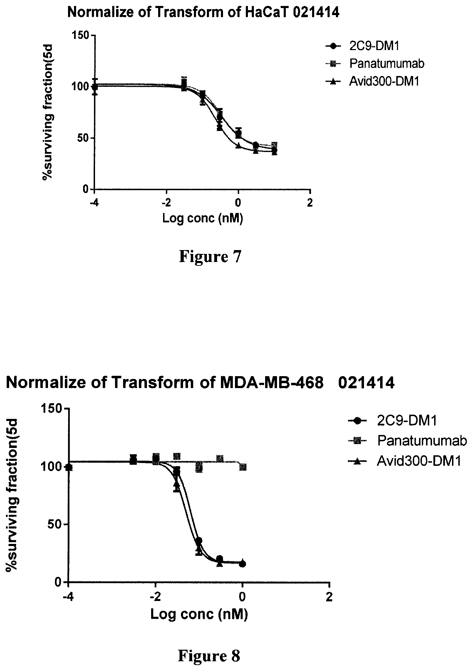

Figure 7 is a graph showing the cytotoxic activity of naked anti-EGFR antibody panitumumab and maytansinoid conjugates thereof against primary keratinocytes. 2-3,000 cells/well were seeded in a flat bottom 96-well tissue culture plate, and incubated with various concentrations of test article in culture media for 72 h or 5 days at 37°C. Viability of the remaining cells was determined via alamar blue cell viability assay.
Figure 8 is a graph showing the cytotoxic activity of naked anti-EGFR antibody panitumumab and maytansinoid conjugates thereof against primary keratinocytes. 2-3,000 cells/well were seeded in a flat bottom 96-well tissue culture plate, and incubated with various concentrations of test article in culture media for 72 h or 5 days at 37°C. Viability of the remaining cells was determined via alamar blue cell viability assay.
Figures 9-13 show results with immunoconjugates that incorporate elements not selected according to the criteria prescribed herein, as discussed in Example 4.
Figure 9 shows that panitumumab and cetuximab are very strong EGFR activation blockers and thus will not be potentiated on normal keratinocytes by conjugation, whereas partially antagonistic antibodies such as J2898A is potentiated (See Figure 10, and 11).

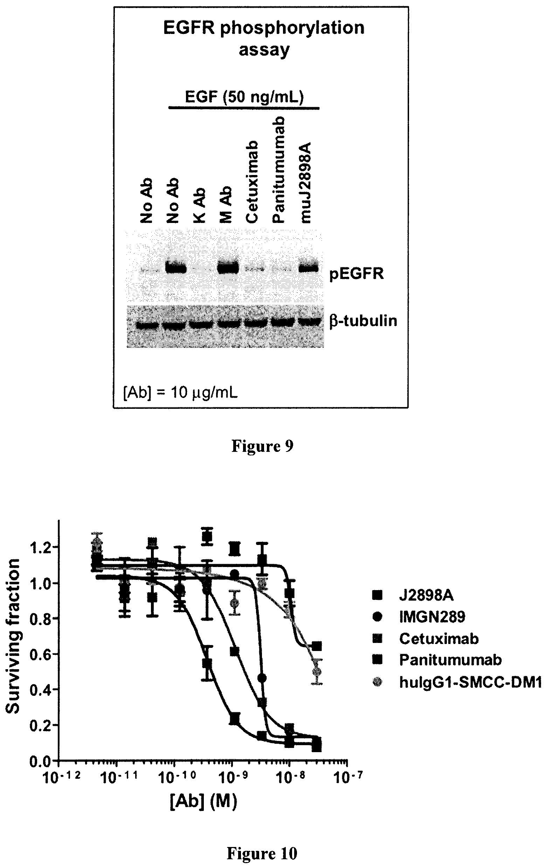

Figure 10 shows that partially antagonistic anti-EGFR antibody by IMGN (J2989A) is potentiated by conjugation (IMGN289) on normal keratinocytes (see also Setiady et al, Proceedings of the 104th Annual Meeting of the American Association for Cancer Research; 2013 Apr 6-10; Washington, DC. Philadelphia (PA): AACR; Cancer Res 2013;73(8 Suppl):Abstract nr 5463).

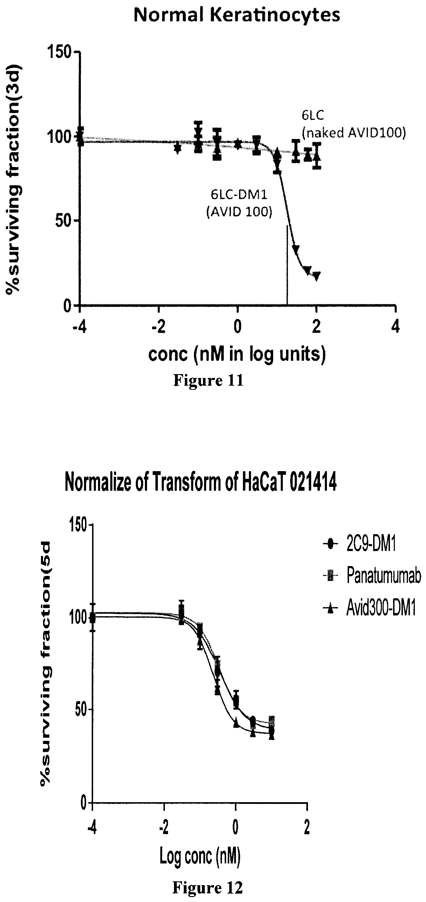

Figure 11 shows the toxicity of cetuximab mutant antibody, 6-LC, (having reduced affinity to make it a partially antagonistic against EGFR) is potentiated by conjugation to payload via non-cleavable linker (6LC-DM1).
Figure 12 shows panitumumab, and panitumumab-DMl effects on keratinocytes, and reveals that conjugation of panitumumab via non-cleavable linker to DM1 (Avid300-DM1) does not increase its activity against normal cells; 2C9-DM1 is cetuximab-DMl conjugate used as a control.

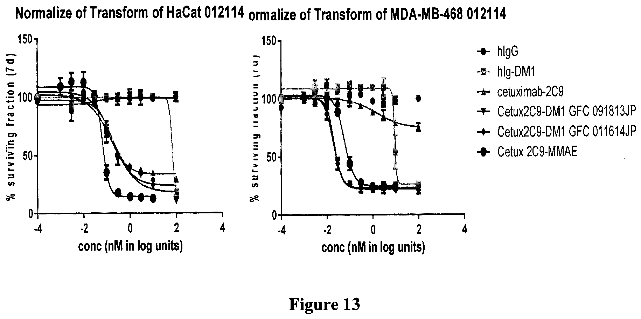

Figure 13 shows that conjugation of cetuximab to MMAE anti-microtubule payload by a cleavable linker (valine-citruline) potentiates its toxicity against both normal cells and MDA-MB-468 cancer cells (Cetux 2C9-MMAE), whereas conjugation via non-cleavable linker (SMCC) only (Cetux2C9-DM1) potentiates anti-cancer activity, thus demonstrating a favorable therapeutic window.

### DETAILED DESCRIPTION OF THE PREFERRED EMBODIMENTS

The present immunoconjugates are based on antibodies that bind to the human epidermal growth factor receptor (hEGFR), a protein that is presented on the surface of many different cell types including particularly skin cells such as keratinocytes. As used herein, the term "hEGFR" refers to any protein that comprises the expressed and processed product of the human *her-1* gene, wherein the protein is designated as UniProtKB/Swiss-Prot P00533. The term EGFR is used generically herein, and refers to the wild type protein and all naturally occurring variants thereof. The term "wtEGFR" is used more specifically with reference only to the wild type form of human EGFR. The term "EGFRvIII" refers to the EGFR variant protein that comprises the expressed and processed product of a variant of the *her-1* gene lacking exons 2-7, and thus includes only the polypeptide sequence encoded by exons 1 and 8 of *her-1.* The term "domain III" is not related to EGFRvIII, and instead refers to a location within EGFR, and represents an extracellular site that is key for EGF ligand binding, and binding of highly antagonistic antibodies cetuximab and panitumumab (Voigt et al, 2012 November; 14(11): 1023-1031).

The present immunoconjugates comprise an EGFR antibody that is a full antagonist at the EGFR. An EGFR antibody that is a "full antagonist" is an antibody that blocks completely or nearly so the transmission of a signal that is stimulated, in the normal course, by the EGF ligand through wtEGFR to the wtEGFR-coupled tyrosine kinase. EGFR antibodies that are full antagonists are particularly EGFR antibodies that bind directly to EGFR domain III. EGFR antibodies having these properties and an EGFR binding affinity of 5 nanomolar (nM) or less are particularly preferred for inclusion in the present immunoconjugates. By this measure, and as shown in Figure 9, at least the following known antibodies are not suitable for use in the present immunoconjugates: J2898A, intellimab 6-LC (a cetuximab variant taught in WO 2012/100346), nimotuzumab, and matuzumab.

It is found that when a full antagonist EGFR antibody is selected to deliver a toxic payload, the killing effect is potentiated only on EGFR+ cancer cells and not on EGFR+ normal cells such as keratinocytes. This is not the case when the EGFR antibody is a partial antagonist, i.e., an antibody that allows transmission of some EGF-mediated signal. When conjugated to a toxin, these partial antagonist antibodies show a potentiation of the killing effect at both normal cells that are EGFR+ and cancer cells that are EGFR+.

The present immunoconjugates are based more particularly, and in one embodiment, on the hEGFR antibody known as cetuximab, now commercially available from Eli Lilly and Company under the trade name Erbitux®. Cetuximab is a recombinant, human/mouse chimeric IgG1 antibody that binds specifically to the extracellular domain of wtEGFR. The amino acid sequences of the CDRs for both the heavy chain of cetuximab (SEQ ID Nos.1-3) and the light chain of cetuximab (SEQ ID Nos. 4-6) are listed herein. Also listed are the amino acid sequences of the heavy chain variable region (SEQ ID No.7) and of the light chain variable region (SEQ ID No.8) of cetuximab, together with the amino acid sequences of the complete heavy chain (SEQ ID No. 9) and complete light chain (SEQ ID No. 10) of cetuximab. According to the invention, the EGFR antibody has the amino acid sequence of SEQ ID No. 10 for the light chain and SEQ ID No. 9 for the heavy chain. Cetuximab variants useful herein are highly antagonistic EGFR-binding agents that compete with cetuximab for binding to human EGFR. Useful cetuximab variants have been mentioned hereinabove, and include fragments of cetuximab comprising the EGFR binding sites of cetuximab, such as all of the light chain and heavy chain CDRs herein recited. Other cetuximab variants useful here in are cetuximab variants that incorporate one, two or more substitutions outside the antigen binding domains, such as in the framework region or in the constant region (Fc). Such substitutions are benign in the sense that they do not reduce cytotoxicity relative to cetuximab per se.

In another embodiment of the disclosure, the present immunoconjugates are based on the EGFR antibody known as panitumumab, now commercially available and sold under the trade name Vectibix®. Panitumumab is a recombinant, fully human IgG2 antibody that binds specifically to the extracellular domain of wtEGFR. The amino acid sequences of the heavy and light chains of panitumumab are listed in US 6,235,883 and US 7,807,798. A useful alternative to panitumumab is an EGFR binding variant thereof that competes with panitumumab for EGFR binding, such as a fragment of panitumumab that incorporates its antigen binding sites but has an otherwise lost or altered constant region.

The present immunoconjugates of the disclosure can also be based on still other EGFR antibodies provided they show full antagonist activity as defined above, such as EGFR antibodies that bind selectively to domain III of EGFR, and any other EGFR antibodies that compete with EGF and block fully or nearly so the transmission of EGF-stimulated downstream signalling.

In the present immunoconjugates, the full antagonist EGFR antibody, such as cetuximab, is conjugated to an anti-microtubule toxin such as maytansinoid toxin. By "anti-microtubule toxin" is meant an agent having cell toxicity mediated by interference with the microtubule structures important for cell mitosis, such as by inhibiting the formation of tubulin or by inhibiting the organization thereof.

Included within this toxin family are the maytansinoids and auristatins, and many other agents developed more recently and having the same mechanism of action. The auristatins in particular block cell replication by inhibiting polymerization of tubulin and are thus anti-mitotic. The structure of an auristatin useful herein and known as MMAE, or vedotin, is shown below:

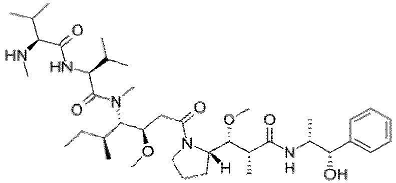

There are various forms of maytansinoids that are useful. These are all based on the complex structure of a natural molecule, maytansine.

Particularly useful are the maytansinoids including DM-1 and DM-4. According to the invention, the toxin coupled to the EGFR MAb is DM-1 having the structure shown *infra.*

Also useful as anti-microtubule toxins are dolostatins, auristatins, tubulysins and cryptophycins. Specific examples of useful species within each genus include dolostatin 10, mono methyl dolostatin 10, auristatin E, mono methyl auristatin E (MMAE), auristatin F, monomethyl auristatin F, HTI-286, tubulysin M, as well as the tubulin binders such as tubulysin IM-1, tubulysin IM-2, tubulysin IM-3, colchicine DA, and maytansinoids AP-3, DM-1 and DM-4.

Conjugates of a full antagonist EGFR antibody such as cetuximab, and an anti-microtubule toxin such as a maytansinoid or auristatin can be formed using any technique presently known or later developed that couples a linker that is "non-cleavable". These linkers remain intact, and retain the antibody and toxin in covalent association, throughout conditions normally encountered following administration to a subject, including extracellular environments. More specifically, non-cleavable linkers result in ADC constructs for which release of the cytotoxic payload is achieved by destruction of the antibody by intracellular lysosomes.

Methods of linker integration are described for instance in US 5,208,020; US 8,088,387; and US 6,441,163. A preferred method is to modify the EGFR antibody, e.g., cetuximab, with succinimidyl 4-(N-maleimidomethyl)-cyclohexane-1-carboxylate (SMCC) to introduce maleimido groups followed by reaction of the modified antibody with a thiol-containing maytansinoid to give a thioether-linked conjugate. The resulting chemical structures are shown below. Conjugates with 1 to 10 drug molecules per antibody molecule result.

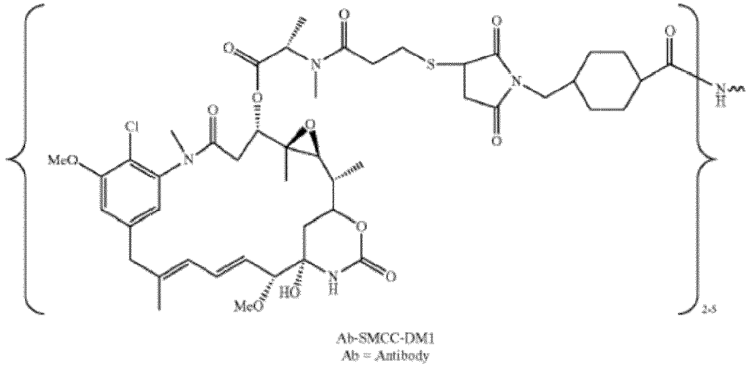

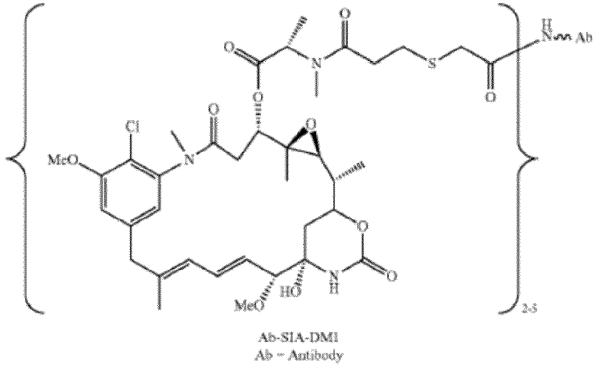

Other useful forms of non-cleavable linkers include N-Succinimidyl iodoacetate (SIA), sulfo-SMCC, m-maleimidobenzoyl-N-hydroxysuccinimide ester(MBS), sulfo-MBS and succinimidyl-iodoacetate, as described in the literature, to introduce 1-10 reactive groups. (see, Yoshitake et al, 101 Eur. J. Biochem. 395-399 (1979); Hashida et al, J. Applied Biochem. 56-63 (1984); and Liu et al, 18 690-697 (1979)). Particularly useful for linking auristatins as anti-microtubule toxin are the non-cleavable maleimidocaproyl linkers described by Doronina et al, in Bioconjugate Chem., 2006 Jan-Feb;17(1):114-24).

According to the invention, the immunoconjugate comprises an EGFR antibody having the amino acid sequence of SEQ ID NO: 10 for the light chain and SEQ ID NO: 9 for the heavy chain linked to DM-1 by an SMCC linker.

In another specific embodiment of the disclosure, the immunoconjugate comprises panitumumab linked to DM-1 by an SMCC linker.

Thus, in a general aspect, the disclosure provides a method useful to potentiate the anti-cancer activity of an EGFR antibody without potentiating the effect thereof on normal EGFR+ cells, the method comprising:
(i) selecting, for conjugation, an EGFR antibody that is a full EGFR antagonist;
(ii) selecting, for delivery by the EGFR antibody, an anti-microtubule toxin;
(iii) selecting, for coupling the selected EGFR antibody to the anti-microtubule toxin, a linker that is non-cleavable; and
incorporating the linker between the antibody and the toxin to provide an immunoconjugate having a cytotoxic activity that is potentiated against EGFR+ disease cells but not against normal EGFR+ cells.

As will be appreciated from the preceding disclosure, the full antagonist EGFR antibody according to the invention has the amino acid sequence of SEQ ID NO: 10 for the light chain and SEQ ID NO: 9 for the heavy chain. The anti-microtubule toxin is preferably an auristatin or a maytansinoid, and is according to the invention DM-1. The non-cleavable linker is according to the invention SMCC.

In another specific aspect of the disclosure, there is the proviso that the full antagonist EGFR antibody is not cetuximab. In a further specific embodiment, there is the proviso that the full antagonist EGFR antibody is not panitumumab.

Therapeutic formulations of the conjugate are prepared for therapeutic use directly or for storage by mixing the conjugate having the desired degree of purity with optional pharmaceutically acceptable carriers, excipients or stabilizers (Remington's Pharmaceutical Sciences, 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl, or benzyl alcohol; alkyl parabens such as methyl or propyl paraben; catechol; resorcinol; cyclohexanol; 3-pentanol; and m-cresol); low molecular weight (less than about 10 residues) polypeptides; proteins such as serum, albumin, gelatin, or immunoglobulins; hydrophilic polymers such as polyvinylpyrrolidone; amino acids such as glycine, glutamine, asparagines, histidine, arginine or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN, PLURONICS or polyethylene glycol (PEG).

The active ingredients to be used for *in vivo* administration must be sterile. This is readily accomplished by filtration through sterile membranes.

Sustained-release preparations may be prepared. Suitable examples of sustained-release include semipermeable matrices of solid hydrophobic polymers containing the conjugate, which matrices are in the form of shapes articles, e.g., films or microcapsules. Examples of sustained-release matrices include polyesters, hydrogels (for example, poly (2-hydroxyethyl-methacrylate), polylactides (U.S. Pat. No. 3,773,919), copolymers of L-glutamic acid and ethyl-L-glutamate, non-degradable ethylene-vinyl acetate, degradable lactic acid-glycolic acid copolymers such as injectable microspheres composed of lactic acid-glycolic acid copolymer and leuprolide acetate, and poly-D-(-)-3-hydroxybutyric acid. While polymers such as ethylene-vinyl acetate and lactic acid-glycolic acid enable release of molecules for over 100 days, certain hydrogels release proteins for shorter time periods.

The conjugates are useful to treat EGFR+ disease cells. Such treatment results in a reduction in the number, size or distribution of such disease cells in subjects presenting with them. In embodiments, the conjugates are used to treat EGFR+ disease cells that are EGFR+ cancer cells and tumours comprising them. Such treatment results preferably in a reduction in the number, size, volume or distribution of such cancer cells and tumours comprising them, or at least in a reduction in the rate at which such disease cells increase in number, size, volume or distribution of such cells and tumours in subjects presenting with them.

Subjects presenting with EGFR+ cancer cells can be identified with the aid of assays that detect the receptor, as protein or as nucleic acid precursor (DNA or RNA) in physiological samples such as biopsied tissue. A suitable test for EGFR protein is the commercially available and FDA approved Dako EGFR pharmDx® test kit.

For the treatment of subjects presenting with EGFR+ cancer cells, the appropriate dosage of the conjugate will depend on the type of disease to be treated, as defined above, the severity and course of the disease, whether the agent is administered for preventative or therapeutic purposes, previous therapy, the patients clinical history and response to the agent, and the discretion of the attending physician. The agent is suitably administered to the patient at one time or over a series of treatments.

For example, depending on the type and severity of the disease, about 1 µg/kg to 15 mg/kg (e.g., 0.1-20 mg/kg) of conjugate is a candidate dosage for administration to the patient, whether, for example, by one or more separate administrations, or by continuous infusion. A typical daily dosage might range from about 1 µg/kg to 500 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays. It will thus be appreciated that an effective amount of the immunoconjugate is an amount effective alone or as part of a treatment regimen that retards or inhibits the rate of growth or proliferation of EGFR+ disease cells.

An EGFR+ disease cell is a disease cell that presents EGFR on its surface as detectable for instance by EGFR antibody binding, or by detection of intracellular mRNA encoding *her-1.* Particular EGFR+ disease cells include those having on their surface an abnormally high density and/or activity of EGFR molecules, or the presence of the EGFRvIII variant of EGFR.

It may be useful to administer the present conjugates by intravenous infusion first as loading dose, followed by maintenance dose, such as at an initial dose of 4mg/kg over 90 minutes, then 2 mg/kg over 30 minutes, once weekly for as many as 52 weeks, with follow up as required. In the specific case of the panitumumab conjugate, dosing might be based on that utilized for panitumumab per se, which comprises 6mg/kg given once every two weeks as a one hour infusion.

The conjugates are useful in the treatment of a variety of cancers, to inhibit the growth or proliferation of EGFR+ cancer cells and tumours comprising them, including hematopoietic cell cancers and solid tumours. Conditions or disorders to be treated include benign or malignant tumors (e.g., renal, liver, kidney, bladder, breast, gastric, ovarian, colorectal, prostate, pancreatic, lung, vulva, and thyroid); hepatic carcinomas; sarcomas; glioblastomas; and various head and neck tumors; leukemias and lymphoid malignancies. In particular embodiments, the antibody or bivalent fragment are used in the treatment of such cancer cells that express EGFRvIII, as determined by the screening assays herein described. In particular embodiments, the cancer cells are EGFR+-presenting cancer cells that include head and neck cancers and especially squamous cell carcinoma of the head and neck, colorectal cancers, gastrointestinal cancers, brain tumours including glioblastomas, and tumours of the lung including non-small-cell lung carcinoma, and of the breast, pancreas, esophagus, kidney, ovary, cervix and prostate. In specific embodiments, the EGFR+ cancer is one for which cetuximab has received FDA marketing approval, such as squamous cell carcinoma of the head and neck and colorectal cancers.

It will be appreciated that subjects who could benefit from the present method include mammals including humans as well as livestock, and pets.

Other therapeutic regimens may be combined with the administration of the conjugates of the instant invention. For example, the patient to be treated may also receive radiation therapy, such as external beam radiation. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M. C. Perry, Williams & Wilkins, Baltimore, Md. (1992). The chemotherapeutic agent may precede, or follow administration or the conjugate or may be given simultaneously therewith. The conjugate may be combined with any anti-cancer toxins, or any other suitable drug particularly including irinotecan (CPT-11), cisplatin, cyclophosphamide, melphalan, dacarbazine, doxorubicin, daunorubicin, and topotecan, as well as tyrosine kinase inhibitors, including particularly EGFR kinase inhibitors such as AG1478 ((4-(3-chloroanilino-6,7-dimethoxyquinazoline), gefitinib (Iressa®), erlotinib (Tarceva®), lapatinib (Tykerb®), canertinib (PD183805, Pfizer), PKI-166 (Novartis), PD158780 and pelitinib.

It may also be desirable to administer antibodies or conjugates against other tumor associated antigens or their ligands, such as antibodies which bind to the ErbB2 (including trastuzumab marketed as Herceptin®, and pertuzumab marketed as Omnitarg®), ErbB3, ErbB4, or vascular endothelial factor (VEGF), and/or antibodies that bind to EGF or TGFα.

In another aspect of the disclosure, an article of manufacture containing conjugate in an amount useful for the treatment of the disorders described herein is provided. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or vial having a stopper pierceable by a hypodermic injection needle). The label on or associated with the container indicates that the composition is used for treating a cancer condition. The article of manufacture may further compromise a second container compromising a pharmaceutically-acceptable buffer, such as phosphate-buffered saline, Ringer's solution and dextrose solution. It may further include other matters desirable from a commercial and use standpoint, including other buffers, diluents, filters, needles, syringes, and package inserts with instructions for use.

An anti-cancer immunoconjugate according to the invention may be for administration with a pharmaceutically-acceptable diluent, carrier, or excipient, in unit dosage form. Unit doses of the conjugate are suitably 50mgs, 100mgs, 150mgs, 200mgs, 250mgs, 300mgs and 400mgs. The drug can be formulated in single use vials at a concentration such as 20mg/mL, for instance 100mg in 5mL vehicle such as 0.9% saline, 200mg in 10mL or 400mg in 20mL.

Any appropriate route of administration can be employed, for example, parenteral, intravenous, subcutaneous, intramuscular, intracranial, intraorbital, ophthalmic, intraventricular, intracapsular, intraspinal, intracisternal, intraperitoneal, intranasal, aerosol, pulmonary, or oral administration.

### Example 1 - Preparation of Cetuximab-SMCC-DMl conjugate (A-H)

### A. PREPARATION AND MEASUREMENT OF CETUXIMAB ANTIBODY

Cetuximab is obtained from the open market, or is produced as described in WO 2012/100346, for conjugation to DM1 using the non-cleavable heterobifunctional cross-linking reagent SMCC.

Cetuximab antibody was then buffer exchanged into 50 mM potassium phosphate, 50 mM sodium chloride, 2 mM EDTA; pH 6.5 buffer (Buffer A). All buffers in this experiment were tested to be free of endotoxin using a chromogenic Limulus amoebocyte lysate (LAL) method (Cambrex). The concentration of antibody was measured using an extinction coefficient of 1.45 mL/mg/cm at 280 nm and a molecular weight of 145,781g.

### B. PREPARATION AND MEASUREMENT OF SMCC STOCK SOLUTION

A 20 mM solution of SMCC (6.69 mg/mL) (Concortis Biosystems Corp.) was prepared in DMSO. The solution was diluted 1/40 in Assay Buffer and the absorbance of the samples was measured at 302 nm. The concentration of the stock solution was calculated using a molar extinction coefficient of 602/M/cm.

### C. PREPARATION AND MEASUREMENT OF DM1 STOCK SOLUTION

A 10 mM solution of DM1 (free thiol form; Concortis Biosystems Corp.) was prepared in DMA (7.37 mg/mL). The absorbance of dilutions of the stock solution in ethanol was measured at 280 nm. The concentration of stock DM1 was calculated by using a molar extinction coefficient of 5700/M/cm at 280 nm. The concentration of free -SH in the stock DM1 preparation was measured using Elman's reagent (DTNB). Dilutions of the stock solution were prepared in Assay buffer made to 3% (v/v) DMA, and then 100 mM DTNB in DMSO (1/100th volume) was added. The increase in absorbance at 412 nm was measured against a reagent blank and the concentration was calculated using an extinction coefficient of 14150/M/cm. The concentration of -SH resulting from the Elman's assay was used to represent the DM1 stock concentration in calculations for conjugation conditions.

### D. MODIFICATION OF CETUXIMAB WITH SMCC CROSSLINKER

The antibody was modified using a 7.5-fold molar excess of SMCC at 20 mg/mL antibody. The reaction was carried out in Buffer A (95% v/v) with DMSO (5% v/v) for 2 hours at room temperature with stirring.

### E. G25 CHROMATOGRAPHY TO REMOVE EXCESS SMCC

The cetuximab-SMCC reaction mixture was gel-filtered through a 1.5×4.9 cm pre-packed column of Sephadex G25 resin equilibrated in Buffer A. The load and elution volumes were according to manufacturer's instructions (Amersham Biosciences). The concentration of the modified antibody solution was assayed spectrophotometrically using the extinction co-efficient described above.

### F. CONJUGATION OF CETUXIMAB-SMCC WITH DM1

The modified antibody was reacted with a 1.7-fold excess of DM1 over linker (assuming 5 linkers per antibody). The reaction was carried out at 10 mg/mL antibody concentration in Buffer A (94% v/v) with DMA (6% v/v). After addition of DM1, the reaction was incubated at room temperature for 16.5 hours with stirring.

### G. CONJUGATION PURIFICATION BY G25 CHROMATOGRAPHY

The conjugation reaction mixture was gel-filtered through a 1.5×4.9 cm pre-packed column of Sephadex G25 resin equilibrated in 1 × phosphate buffered saline (PBS), pH 6.5 (Buffer B). The load and elution volumes were according to manufacturer's instructions (Amersham Biosciences). The number of DM1 molecules linked per mole of cetuximab was determined by measuring absorbance at both 252 nm and 280 nm of the eluted material. The DM1/antibody ratio was found to be 2 and 4. The resulting conjugate was analyzed for binding and cytotoxicity.

### H. TESTING OF CETUXIMAB-SMCC-DM1

The cell lines used in these studies have the following characteristics:
A431: human epithelial carcinoma of vulva cell line; available from ATCC; plated at 4000 cells/well in DMEM-10%FBS, 100µl/well in 96 well plate.
H226: lung squamous cell carcinoma cell line; available at ATCC; plated at 4000 cells/well in RPMI-10%FBS, 100µl/well in 96-well culture plate.
MDA-MB-468: mammary gland/breast; derived from metastatic site: pleural effusion; available from ATCC; plated at 4000 cells/well in ATCC-formulated Leibovitz's L-15 Medium (Catalog No. 30-2008) with added fetal bovine serum to a final concentration of 10%; 100µl/well in 96 well plate.
HaCaT: in vitro spontaneously transformed keratinocytes from histologically normal skin; available from Chinese Center for Type Culture Collection of Wuhan University; plated at 2000 cells/well in DMEM-10%FBS, 100µl/well in 96-well culture plate.
HEKa: Normal Human Primary Epidermal Keratinocytes, adult; available from PromoCell GmbH; plated at 4000cells/well in EpiLife media 100µl/well, EpiLife medium #MEP1500CA, Invitrogen +HKGS human keratinocyte growth supplement (#S-001-5)

Binding studies showed that the conjugation of antibody to DM1 did not affect the K_{D}; both naked cetuximab antibody and cetuximab-SMCC-DMl conjugate had the same binding affinity to EGFR by surface plasmon resonance (∼0.3 nM, Table 1). Evaluation of the *in vitro* cytotoxicity of the sample showed that the cetuximab-SMCC-DMl conjugate is significantly more cytotoxic compared to cetuximab against cancer cell lines (Figs 3&4), but that surprisingly cetuximab-SMCC-DMl and naked cetuximab have the same cytotoxic activity against keratinocytes (Figs 5&6).

**Table 1: effects of conjugation to DM1 on cetuximab KD**

| Sample | # of experiments | EGFR ecd K_{D} (nM) |
|---|---|---|
| Commercial cetuximab | 3 | 0.27 +/-0.02 |
| Cetuximab-SMCC-DM1 | 3 | 0.28 +/-0.02 |

The effect of DM1 conjugation on different EGFR MAbs (huML66 and huEGFR-7R) is demonstrated in ImmunoGen's published US patent application 2012/0156217, and Figures 1 and 2 herein are reproductions of figures appearing in that application. More particularly, as shown in Figure 1, conjugation of anti-EGFR antibodies huML66-SMCC-DM1 and huEGFR-7R to maytansinoids to create huML66-SMCC-DM1 and huEGFR-7R-SMCC-DM1 immunoconjugates increases cytotoxic activity of against normal keratinocytes. Similarly, and as expected, conjugation of anti-EGFR antibodies huML66 and huEGFR-7R to maytansinoids to create huML66-SMCC-DM1 and huEGFR-7R-SMCC-DM1 immunoconjugates significantly potentiates cytotoxic activity of the antibodies against H226 cancer cell line (Figure 2).

As shown in Figures 3 and 4, DM1 conjugation of anti-EGFR antibody cetuximab (HC-LC) to create cetuximab-SMCC-DMl immunoconjugate (HC-LC/DM1) significantly potentiates cytotoxic activity of the antibody against cancer cells, as expected and shown in Figure 3. As shown in Figure 4, and also as expected, conjugation of anti-EGFR antibody cetuximab (HC-LC) to maytansinoids to create cetuximab-SMCC-DM1 immunoconjugate (HC-LC/DM1) significantly potentiates cytotoxic activity of the antibody against cancer cells.

However, and quite surprisingly, as shown in Figures 5 and 6, conjugation of anti-EGFR antibody cetuximab (HC-LC) to maytansinoids to create cetuximab-SMCC-DM1 immunoconjugate (HC-LC/DM1) did not increase cytotoxicity against the keratinocytes as is reflected in substantially the same IC₅₀ values for these agents (IC₅₀ of 0.8269 for conjugate vs. IC₅₀ of 0.4324 for naked antibody; Fig 5, HaCaT - normal human keratinocytes cell line & IC₅₀ of 2.210 for conjugate vs. IC₅₀ of 0.9348 for naked antibody, Fig 6; HEKa- primary human keratinocyte cells). As used herein, IC₅₀ refers to the concentration of drug required to kill 50% of cells.

The measure of whether cytotoxicity is increased or not is determined above by comparing IC₅₀ values produced using the alamar blue cell viability assay (each experiment performed in triplicates in a 96-well plate). A difference within one log order is deemed not significant and is considered to reveal no change in cytotoxicity, whereas a difference greater than one log order reveals a significant change in cytotoxicity.

### Example 2 - Evaluation in Primates

Cynomolgus monkey species has been previously demonstrated to be a valuable and highly predictive model for evaluating anti-EGFR antibodies toxicities, including dermatologic side-effects. The high level of correlation between Cynomolgus monkey and human toxicity data for EGFR-targeting antibodies is in part due high homology between the monkey and human EGFR receptors that results in very similar K_{D} values for the antibodies:

**Table 1:** Binding affinity of cetuximab and panitumumab to human and cynomologus EGFR is consistent across the species. Apparent antibody affinity defined as the antibody concentration (picomolar, pM) required to achieve half-maximal binding in ELISA with recombinant extracellular domains of human EGFR (huEGFR), and Cynomolgus monkey EGFR (cyEGFR).Adapted from Koefoed et al, MABs. 2011 Nov-Dec; 3(6):584-595.

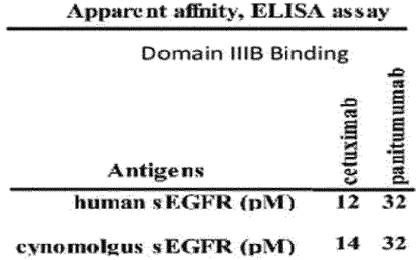

The cetuximab-SMCC-DM1 immunoconjugate was tested in primates particularly to identify any significant potentiation of antibody side-effects by the conjugated toxin on normal cells. In particular, close attention was paid to dermatologic side-effects that are qualitatively different and/or significantly exacerbated relative to what is expected with the naked fully antagonistic anti-EGFR antibody.

The study initially made use of two Cynomolgus macaques, all males that were sedated with ketamine and anesthetized with isoflurane to facilitate IV administration of the antibody-drug conjugate administered at 10mg/kg. Animals were then observed for 5 days prior to sacrifice for general signs of acute toxicity, and examined further.

The detailed histopathology analysis revealed no severe dermatologic toxicities or other severe and qualitatively new dermatologic side-effects other than those that are expected as a result of naked cetuximab treatment. Also, no other severe on-target toxicities were observed in these animals demonstrating safety of ADCs of present invention and absence of significant exacerbation of the naked antibody toxicities.

Subsequent to successful completion of the acute primate study, repeat-dose safety study has been initiated in additional animals. The ADC was administered once every three weeks at 10mg/kg dose over 4 cycles. This dose and schedule was selected based on dosing schedules commonly used with other ADCs in primates and humans (Poon KA et al, Preclinical safety profile of trastuzumab emtansine (T-DM1): mechanism of action of its cytotoxic component retained with improved tolerability. Toxicol Appl Pharmacol. 2013 Dec 1;273(2):298-313; FDA Package Insert for Kadcyla™, accessed February 24, 2014: http://www.accessdata.fda.gov/drugsatfda_docs/label/2013/1254271bl.pdf).

With the exception of reversible liver enzyme elevations which is an expected class side-effect of ADCs, no other severe and/or qualitatively new dermatologic or other on-target side-effects other than those that are already expected as a result of naked cetuximab treatment (cetuximab cynomolgus monkey data available at: http://www.accessdata.fda.gov/drugsatfda_docs/bla/2004/125084_ERBITUX_PHARMR _P3.PDF) have been observed in the ongoing study to date. Severe skin toxicity and toxic epidermal necrolysis previously reported with anti-CD44v6 ADC (which also uses SMCC-DM1 payload technology and targets an antigen expressed by normal keratinocytes) has not been observed in this study (Tijink et al., Clin Cancer Res, 2006, 12:6064). These safety data further validates *in vitro* results that on-target toxicities of fully antagonist naked anti-EGFR antibodies is not significantly potentiated as result of conjugation to the cytotoxic payloads.

### Reference Example 3 - Preparation of Panitumumab-SMCC-DM1 conjugate (A-H)

A DM-1 conjugated panitumumab was prepared essentially as described above for the cetuximab counterpart. More particularly,

### A. PREPARATION AND MEASUREMENT OF PANITUMUMAB ANTIBODY

Panitumumab is obtained from the open market, or is produced as described in US 6,235,883 or US 7,807,798 for conjugation to DM1 using the non-cleavable heterobifunctional cross-linking reagent SMCC.

Panitumumab antibody was then buffer exchanged into 50 mM potassium phosphate, 50 mM sodium chloride, 2 mM EDTA; pH 6.5 buffer (Buffer A). All buffers in this experiment were tested to be free of endotoxin using a chromogenic Limulus amoebocyte lysate (LAL) method (Cambrex). The concentration of antibody was measured using an extinction coefficient of 1.45 mL/mg/cm at 280 nm and a molecular weight of 145,781g.

### B. PREPARATION AND MEASUREMENT OF SMCC STOCK SOLUTION

A 20 mM solution of SMCC (6.69 mg/mL) (Concortis Biosystems Corp.) was prepared in DMSO. The solution was diluted 1/40 in Assay Buffer and the absorbance of the samples was measured at 302 nm. The concentration of the stock solution was calculated using a molar extinction coefficient of 602/M/cm.

### C. PREPARATION AND MEASUREMENT OF DM1 STOCK SOLUTION

A 10 mM solution of DM1 (free thiol form; Concortis Biosystems Corp.) was prepared in DMA (7.37 mg/mL). The absorbance of dilutions of the stock solution in ethanol was measured at 280 nm. The concentration of stock DM1 was calculated by using a molar extinction coefficient of 5700/M/cm at 280 nm. The concentration of free -SH in the stock DM1 preparation was measured using Elman's reagent (DTNB). Dilutions of the stock solution were prepared in Assay buffer made to 3% (v/v) DMA, and then 100 mM DTNB in DMSO (1/100th volume) was added. The increase in absorbance at 412 nm was measured against a reagent blank and the concentration was calculated using an extinction coefficient of 14150/M/cm. The concentration of -SH resulting from the Elman's assay was used to represent the DM1 stock concentration in calculations for conjugation conditions.

### D. MODIFICATION OF PANITUMUMAB WITH SMCC CROSSLINKER

The antibody was modified using a 7.5-fold molar excess of SMCC at 20 mg/mL antibody. The reaction was carried out in Buffer A (95% v/v) with DMSO (5% v/v) for 2 hours at room temperature with stirring.

### E. G25 CHROMATOGRAPHY TO REMOVE EXCESS SMCC

The panitumumab-SMCC reaction mixture was gel-filtered through a 1.5×4.9 cm pre-packed column of Sephadex G25 resin equilibrated in Buffer A. The load and elution volumes were according to manufacturer's instructions (Amersham Biosciences). The concentration of the modified antibody solution was assayed spectrophotometrically using the extinction co-efficient described above.

### F. CONJUGATION OF PANITUMUMAB-SMCC WITH DM1

The modified antibody was reacted with a 1.7-fold excess of DM1 over linker (assuming 5 linkers per antibody). The reaction was carried out at 10 mg/mL antibody concentration in Buffer A (94% v/v) with DMA (6% v/v). After addition of DM1, the reaction was incubated at room temperature for 16.5 hours with stirring.

### G. CONJUGATION PURIFICATION BY G25 CHROMATOGRAPHY

The conjugation reaction mixture was gel-filtered through a 1.5×4.9 cm pre-packed column of Sephadex G25 resin equilibrated in 1 × phosphate buffered saline (PBS), pH 6.5 (Buffer B). The load and elution volumes were according to manufacturer's instructions (Amersham Biosciences). The number of DM1 molecules linked per mole of panitumumab was determined by measuring absorbance at both 252 nm and 280 nm of the eluted material. The DM1/antibody ratio was found to be 2 and 4. The resulting conjugate was analyzed for binding and cytotoxicity.

### H. TESTING OF PANITUMUMAB-SMCC-DM1

The cell lines used in these studies have the following characteristics:
MDA-MB-468: mammary gland/breast; derived from metastatic site: pleural effusion; available from ATCC; plated at 4000 cells/well in ATCC-formulated Leibovitz's L-15 Medium (Catalog No. 30-2008) with added fetal bovine serum to a final concentration of 10%; 100µl/well in 96 well plate.

HaCaT: in vitro spontaneously transformed keratinocytes from histologically normal skin; available from Chinese Center for Type Culture Collection of Wuhan University; plated at 2000 cells/well in DMEM-10%FBS, 100µl/well in 96-well culture plate.

In vitro studies with keratinocytes demonstrate that conjugation of panitumumab to an anti-microtubule toxin via non-cleavable linker does not potentiate the toxicity of the antibody. The resulting ADC has a similar safety profile on keratinocytes as the naked panitumumab, as well as another ADC based on a fully antagonistic antibody, cetuximab. On MDA-MB-468 cancer cells, significant potentiation of anticancer activity of panitumumab is observed as a result of conjugating the antibody to anti-microtubule toxin via non-cleavable linker. Similar to toxicity observations, panitumumab-based ADC was similarly active as cetuximab-based ADC.

### Example 4 - Results with partial antagonist EGFR MAb conjugates

The effect of selecting immunoconjugate components different from those recommended herein is shown in accompanying drawings. Figure 9 shows that a partial antagonist EGFR antibody, designated J2989A has an activity that is potentiated on normal keratinocytes when conjugated to DM-1. Figure 10 shows, similarly, that another partial antagonist antibody, designated 6-LC (a substitution variant of cetuximab having lower relative affinity for EGFR) has an activity that is also potentiated at normal keratinocytes when conjugated to DM-1. Figure 11 shows that the effect of panitumumab of keratinocytes is not potentiated on keratinocytes when conjugated to DM1, while the anti-cancer effect of the conjugate is potentiated dramatically (Figure 12). And, Figure 13 shows that conjugation of cetuximab to MMAE by a cleavable linker (valine-citrulline) potentiates its toxicity against normal cells and MDA-MB-468 cancer cells, whereas conjugation via non-cleavable linker (SMCC) potentiates anti-cancer activity.

Thus, in these studies, only fully antagonistic anti-EGFR antibodies were not potentiated on normal cells by payload conjugation via non-cleavable linkers, whereas toxicity against normal cells of partial antagonist EGFR antibodies was potentiated by the conjugation. Moreover, we subsequently tested the effect on this activity profile of the cleavable vs. non-cleavable linkers, while maintaining payload mechanism of action the same (i.e. anti-microtubule agent). The antibodies were conjugated via cleavable linker to anti-microtubule payload, MMAE as described by Doronina et al, Nature Biotechnology Nov 7, 2003, Vol 21: pp 778-784. Cleavable linker data demonstrate that when the fully antagonistic antibodies are conjugated to their payloads via cleavable linkers, their toxicity against normal cells is potentiated. Thus, a safe anti-EGFR ADC should incorporate a strongly antagonistic anti-EGFR antibody linked to an anti-microtubule payload by a non-cleavable linker.

### Referenced Sequences

| SEQ ID No. | Description |
|---|---|
| 1 | Cetuximab heavy chain CDR1 |
| NYGVH | |
| 2 | Cetuximab heavy chain CDR2 |
| VIWSGGNTDYNTPFTS | |
| 3 | Cetuximab heavy chain CDR3 |
| ALTYYDYEFAY | |
| 4 | Cetuximab light chain CDR1 |
| RASQSIGTNIH | |
| 5 | Cetuximab light chain CDR2 |
| ASESIS | |
| 6 | Cetuximab light chain CDR3 |
| QQNNNWPTT | |
| 7 | Cetuximab heavy chain variable region (V_{H}) |
| QVQLKQSGPGLVQPSQSLSITCTVSGFSLTNYGVHWVRQSPGKGLEWLGVIWSGGNTDYNTPFTS RLSINKDNSKSQVFFKMNSLQSNDTAIYYCARALTYYDYEFAYWGQGTLVTVSA | |
| 8 | Cetuximab light chain variable region (V_{L}) |
| DILLTQSPVILSVSPGERVSFSCRASQSIGTNIHWYQQRTNGSPRLLIKYASESISGIPSRFSGS GSGTDFTLSINSVESEDIADYYCQQNNNWPTTFGAGTKLELK | |
| 9 | Cetuximab complete heavy chain |
| QVQLKQSGPGLVQPSQSLSITCTVSGFSLTNYGVHWVRQSPGKGLEWLGVIWSGGNTDYNTPFTS RLSINKDNSKSQVFFKMNSLQSNDTAIYYCARALTYYDYEFAYWGQGTLVTVSAASTKGPSVFPL APSSKSTSGGTAALGCLVKDYFPEPVTVSWNSGALTSGVHTFPAVLQSSGLYSLSSVVTVPSSSL GTQTYICNVNHKPSNTKVDKRVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHYTQKSLSLSPGK | |
| 10 | Cetuximab complete light chain |
| DILLTQSPVILSVSPGERVSFSCRASQSIGTNIHWYQQRTNGSPRLLIKYASESISGIPSRFSGS GSGTDFTLSINSVESEDIADYYCQQNNNWPTTFGAGTKLELKRTVAAPSVFIFPPSDEQLKSGTA SVVCLLNNFYPREAKVQWKVDNALQSGNSQESVTEQDSKDSTYSLSSTLTLSKADYEKHKVYACE VTHQGLSSPVTKSFNRGE | |

## Claims

1. A method useful to potentiate the cytotoxicity of an EGFR antibody on EGFR+ disease cells without potentiating the cytotoxicity thereof on normal EGFR+ cells, the method comprising:
(i) selecting, for conjugation, an EGFR antibody that is a full EGFR antagonist and competes with cetuximab for binding to EGFR;
(ii) selecting, for delivery by the EGFR antibody, an anti-microtubule toxin;
(iii) selecting, for coupling the selected EGFR antibody and the anti-microtubule toxin, a linker; and
producing an immunoconjugate that incorporates the linker between the antibody and the toxin, thereby providing an immunoconjugate having a cytotoxicity that is potentiated against EGFR+ disease cells and essentially not potentiated against EGFR+ keratinocyte cells; wherein the selected EGFR antibody is an EGFR antibody having the amino acid sequence of SEQ ID No. 10 for the light chain and SEQ ID No. 9 for the heavy chain, the selected anti-microtubule toxin is the maytansinoid DM-1 and the selected linker is succinimidyl 4-(N-maleimidomethyl)-cyclohexane-1-carboxylate (SMCC).

2. An immunoconjugate comprising (i) an EGFR antibody having the amino acid sequence of SEQ ID No. 10 for the light chain and SEQ ID No. 9 for the heavy chain, and (ii) DM-1 conjugated therewith, by (iii) a linker that is SMCC, the immunoconjugate having a cytotoxic effect relative to a naked form of said antibody that is (1) potentiated with respect to EGFR+ cancer cells, and (2) substantially unaltered with respect to EGFR+ keratinocytes.

3. A pharmaceutical composition comprising the immunoconjugate of claim 2 in an amount cytotoxic to EGFR+ disease cells, and a pharmaceutically acceptable carrier.

4. A method for producing an anti-cancer composition, comprising the step of combining a pharmaceutically acceptable carrier, and an immunoconjugate according to claim 2.

5. The immunoconjugate according to claim 2 or the pharmaceutical composition according to claim 3
for use in a method of treating cancer.

6. The immunoconjugate or pharmaceutical composition for use according to claim 5, wherein the cancer comprises EGFR+ cancer cells.

7. The immunoconjugate or pharmaceutical composition for use according to claim 6, wherein the EGFR+ cancer cells are head and neck cancer cells or colorectal cancer cells.

8. A method for potentiating the effect of an EGFR antibody on EGFR+ disease cells without potentiating the effect thereof on normal EGFR+ cells, comprising linking said EGFR antibody to DM-1 by an SMCC linker, wherein the EGFR antibody has the amino acid sequence of SEQ ID No. 10 for the light chain and SEQ ID No. 9 for the heavy chain.

9. An EGFR antibody in a form conjugated with DM-1 via an SMCC linker for use in a method of treating a subject presenting with a tumour that responds to treatment with an EGFR antibody, wherein treatment elicits a EGFR antibody-mediated adverse response by keratinocytes, whereby the tumour response to treatment with the conjugated EFGR antibody is enhanced essentially without enhancing the adverse keratinocyte response to treatment, relative to treatment with naked antibody alone, wherein the EGFR antibody has the amino acid sequence of SEQ ID No. 10 for the light chain and SEQ ID No. 9 for the heavy chain.

## Patentansprüche

1. Verfahren, das nützlich ist zum Potenzieren der Zytotoxizität eines EGFR-Antikörpers bei EGFR+-Erkrankungszellen ohne die Zytotoxizität davon bei normalen EGFR+-Zellen zu potenzieren, wobei das Verfahren Folgendes umfasst:
(i) Auswählen, zum Konjugieren, eines EGFR-Antikörpers, der ein vollständiger EGFR-Antagonist ist und mit Cetuximab um die Bindung an EGFR im Wettbewerb steht;
(ii) Auswählen, zum Liefern durch den EGFR-Antikörper, eines Antimikrotubulus-Toxins;
(iii) Auswählen, zum Koppeln des ausgewählten EGFR-Antikörpers und des Antimikrotubulus-Toxins, eines Linkers; und
Erzeugen eines Immunkonjugats, das den Linker zwischen dem Antikörper und dem Toxin einschließt, wodurch ein Immunkonjugat bereitgestellt wird, das eine Zytotoxizität aufweist, die gegen EGFR+-Erkrankungszellen potenziert ist und die im Wesentlichen nicht gegen EGFR+-Keratinozytenzellen potenziert ist; wobei der ausgewählte EGFR-Antikörper ein EGFR-Antikörper ist, der die Aminosäuresequenz von SEQ ID No. 10 für die Leichtkette und SEQ ID No. 9 für die Schwerkette aufweist, wobei das ausgewählte Antimikrotubulus-Toxin das Maytansinoid DM-1 ist und der ausgewählte Linker Succinimidyl-4-(N-maleimidomethyl)-cyclohexan-1-carboxylat (SMCC) ist.

2. Immunkonjugat, Folgendes umfassend (i) einen EGFR-Antikörper mit der Aminosäuresequenz von SEQ ID No. 10 für die Leichtkette und SEQ ID No. 9 für die Schwerkette und (ii) damit über (iii) einen Linker, der SMCC ist, konjugiertes DM-1, wobei das Immunkonjugat einen zytotoxischen Effekt im Vergleich auf eine reine Form des Antikörpers aufweist, der (1) in Bezug EGFR+-Krebszellen potenziert ist, und (2) in Bezug auf EGFR+-Keratinozyten im Wesentlichen unverändert ist.

3. Pharmazeutische Zusammensetzung, umfassend das Immunkonjugat nach Anspruch 2 in einer Menge, die für EGFR+-Erkrankungszellen zytotoxisch ist, und einen pharmazeutisch unbedenklichen Träger.

4. Verfahren zum Erzeugen einer Antikrebszusammensetzung, umfassend den Schritt des Kombinierens eines pharmazeutisch unbedenklichen Trägers und eines Immunkonjugats nach Anspruch 2.

5. Immunkonjugat nach Anspruch 2 oder pharmazeutische Zusammensetzung nach Anspruch 3 zur Verwendung in einem Verfahren zum Behandeln von Krebs.

6. Immunkonjugat oder pharmazeutische Zusammensetzung zur Verwendung nach Anspruch 5, wobei der Krebs EGFR+-Krebszellen umfasst.

7. Immunkonjugat oder pharmazeutische Zusammensetzung zur Verwendung nach Anspruch 6, wobei die EGFR+-Krebszellen Kopf- und Halskrebszellen oder Kolorektalkrebszellen sind.

8. Verfahren zum Potenzieren der Wirkung eines EGFR-Antikörpers bei EGFR+-Erkrankungszellen ohne die Wirkung davon bei normalen EGFR+-Zellen zu potenzieren, umfassend das Binden des EGFR-Antikörpers an DM-1 durch einen SMCC-Linker, wobei der EGFR-Antikörper die Aminosäuresequenz von SEQ ID No. 10 für die Leichtkette und SEQ ID No. 9 für die Schwerkette aufweist.

9. EGFR-Antikörper in einer über einen SMCC-Linker mit DM-1 konjugierten Form zur Verwendung in einem Verfahren zum Behandeln eines Subjekts, das einen Tumor aufweist, der auf Behandlung mit einem EGFR-Antikörper anspricht, wobei die Behandlung eine EGFR-Antikörper-vermittelte negative Reaktion durch Keratinozyten auslöst, wobei die Tumorreaktion auf die Behandlung mit dem konjugierten EGFR-Antikörper verstärkt wird, im Wesentlichen ohne die negative Keratinozyten-Reaktion auf die Behandlung im Vergleich zu der Behandlung mit Antikörper allein zu verstärken, wobei der EGFR-Antikörper die Aminosäuresequenz von SEQ ID No. 10 für die Leichtkette und SEQ ID NO. 9 für die Schwerkette aufweist.

## Revendications

1. Procédé utile pour potentialiser la cytotoxicité d'un anticorps contre EGFR sur des cellules pathologiques EGFR+ sans potentialiser la cytotoxicité de celui-ci sur des cellules EGFR+ normales, le procédé comprenant :
(i) la sélection, pour conjugaison, d'un anticorps contre EGFR qui est un antagoniste total d'EGFR et entre en compétition avec le cétuximab pour liaison à EGFR ;
(ii) la sélection, pour délivrance par l'anticorps contre EGFR, d'une toxine antimicrotubule ;
(iii) la sélection, pour couplage de l'anticorps contre EGFR et de la toxine antimicrotubule sélectionnés, d'un lieur ; et
la production d'un immunoconjugué qui incorpore le lieur entre l'anticorps et la toxine, de façon à produire un immunoconjugué ayant une cytotoxicité qui est potentialisée contre des cellules pathologiques EGFR+ et pratiquement pas potentialisée contre des cellules de kératinocytes EGFR+ ; dans lequel l'anticorps contre EGFR sélectionné est un anticorps contre EGFR ayant la séquence d'acides aminés de SEQ ID N° 10 pour la chaîne légère et SEQ ID N° 9 pour la chaîne lourde, la toxine antimicrotubule sélectionnée est le maytansinoïde DM-1 et le lieur sélectionné est le 4-(N-maléimidométhyl)-cyclohexane-1-carboxylate de succinimidyle (SMCC).

2. Immunoconjugué comprenant (i) un anticorps contre EGFR ayant la séquence d'acides aminés de SEQ ID N° 10 pour la chaîne légère et SEQ ID N° 9 pour la chaîne lourde, et (ii) DM-1 conjugué à celui-ci, par (iii) un lieur qui est SMCC, l'immunoconjugué ayant un effet cytotoxique par rapport à une forme nue dudit anticorps qui est (1) potentialisé vis-à-vis de cellules cancéreuses EGFR+, et (2) sensiblement inchangé vis-à-vis de kératinocytes EGFR+.

3. Composition pharmaceutique comprenant l'immunoconjugué selon la revendication 2 en une quantité cytotoxique pour des cellules pathologiques EGFR+, et un véhicule pharmaceutiquement acceptable.

4. Procédé de production d'une composition anticancéreuse, comprenant l'étape de combinaison d'un véhicule pharmaceutiquement acceptable, et d'un immunoconjugué selon la revendication 2.

5. Immunoconjugué selon la revendication 2 ou composition pharmaceutique selon la revendication 3 pour utilisation dans un procédé de traitement du cancer.

6. Immunoconjugué ou composition pharmaceutique pour utilisation selon la revendication 5, le cancer comprenant des cellules cancéreuses EGFR+.

7. Immunoconjugué ou composition pharmaceutique pour utilisation selon la revendication 6, les cellules cancéreuses EGFR+ étant des cellules de cancer de la tête et du cou ou des cellules de cancer colorectal.

8. Procédé pour potentialiser l'effet d'un anticorps contre EGFR sur des cellules pathologiques EGFR+ sans potentialiser l'effet de celui-ci sur des cellules EGFR+ normales, comprenant la liaison dudit anticorps contre EGFR à DM-1 par un lieur SMCC, dans lequel l'anticorps contre EGFR a la séquence d'acides aminés de SEQ ID N° 10 pour la chaîne légère et SEQ ID N° 9 pour la chaîne lourde.

9. Anticorps contre EGFR sous une forme conjuguée avec DM-1 par l'intermédiaire d'un lieur SMCC pour utilisation dans un procédé de traitement d'un sujet présentant une tumeur qui répond au traitement avec un anticorps contre EGFR, le traitement induisant une réponse indésirable médiée par un anticorps contre EGFR par des kératinocytes, la réponse tumorale au traitement avec l'anticorps contre EGFR conjugué est activée essentiellement sans activer la réponse indésirable de kératinocytes au traitement, par rapport au traitement avec un anticorps nu seul, l'anticorps contre EGFR ayant la séquence d'acides aminés de SEQ ID N° 10 pour la chaîne légère et SEQ ID N° 9 pour la chaîne lourde.
